# EUROPEAN PATENT APPLICATION

(11) **EP 4 043 874 A1**
(43) Date of publication of application: **17.08.2022**
(21) Application number: 20875535.5
(22) Date of filing: 07.10.2020
(51) Int. Cl.: G01N 27/30, G01N 27/416

(54) **ELECTROCHEMICAL SENSOR**

(30) Priority: 09.10.2019 JP 2019186196
(71) Applicant: Showa Denko Materials Co., Ltd., Tokyo 100-6606 (JP); National University Corporation Hokkaido University, Sapporo-shi, Hokkaido 060-0808 (JP)
(72) Inventor: JOHMEN, Masayoshi, Tokyo 100-6606 (JP); OBATA, Kazuhito, Tokyo 100-6606 (JP); SETOGUCHI, Shinichi, Tokyo 100-6606 (JP); KAWAGUCHI, Toshikazu, Sapporo-shi, Hokkaido 060-0808 (JP); ITO, Ryusei, Sapporo-shi, Hokkaido 060-0808 (JP); GUIZANI, Mokhtar, Sapporo-shi, Hokkaido 060-0808 (JP)
(74) Representative: Berggren Oy
(86) International application number: PCT/JP2020/038018
(87) International publication number: WO 2021/070870

(57) **Abstract**

An electrochemical sensor is used in a state of being immersed in water to be inspected for water quality inspection. The electrochemical sensor includes a working electrode, a reference electrode, a first counter electrode, and a second counter electrode. The working electrode, the reference electrode, the first counter electrode, and the second counter electrode are electrically isolated from each other.

## Description

### Technical Field

The present disclosure relates to an electrochemical sensor. The present application claims priority from Japanese Patent Application No. 2019-186196, filed on October 9, 2019, the entire disclosure of which is incorporated herein by reference.

### Background Art

Patent Literature 1 discloses a technology related to a flat plate type three-electrode electrochemical sensor. This electrochemical sensor includes an insulating substrate; and a reference electrode, a working electrode, and a counter electrode, which are provided to be exposed on the surface of the substrate. Wiring pattern to each electrode is embedded in the substrate. The reference electrode is composed of a main part made of gold (Au); and a polyaniline film covering the main part. The working electrode is composed of a main part made of gold; and a ferrocenyl hexanethiol film, which is a self-assembled monolayer film covering the main part. The polyaniline film is formed, after the main part is irradiated with vacuum ultraviolet radiation, by performing a constant current electrolysis method.

### Citation List

### Patent Literature

Patent Literature 1: Japanese Unexamined Patent Publication No. 2015-190811

### Summary of Invention

### Technical Problem

When the water quality is inspected by electrochemical measurement, an electrochemical sensor having a plurality of electrodes is used. By measuring the potential difference, the current, or the alternating current impedance between electrodes in a state in which this electrochemical sensor is immersed in the water to be inspected, the concentration of a substance included in the water to be inspected is measured. In many cases, the multiple electrodes are three electrodes, namely, a working electrode, a reference electrode, and a counter electrode. The working electrode is sensitive to the substance. The reference electrode is not sensitive to the substance and maintains a constant potential. The counter electrode sets a potential difference with the working electrode or sends and receives an electric current to and from the working electrode. Regarding the control system for such an electrochemical sensor, there are a potential control system and a current control system. In the potential control system, a potentiostat is used. The potentiostat applies a voltage between the working electrode and the counter electrode and controls the potential between the working electrode and the reference electrode to a desired value. In the current control system, a galvanostat is used. The galvanostat controls the current between the working electrode and the counter electrode and measures the potential between the working electrode and the reference electrode.

In the water quality measurement using such an electrochemical sensor, it is important to increase the measurement accuracy. For example, with regard to the measurement of the total bacterial count of bacteria, including Escherichia coli and the like, included in the water to be inspected, the background may not be stable while noise may become excessive in the measurement using conventional electrochemical sensors, and practical measurement accuracy may not be obtained. For the measurement of the total bacterial count as described in official methods, methods including culture which is time-consuming method are used, and it is difficult to obtain the results for water quality inspection immediately. An object of the present disclosure is to provide an electrochemical sensor that enables constant monitoring of water quality and enables highly accurate water quality monitoring.

### Solution to Problem

In order to solve the above-described object, an electrochemical sensor according to an embodiment is an electrochemical sensor that is used in a state of being immersed in the water to be inspected for water quality inspection, and the electrochemical sensor includes a working electrode, a reference electrode, a first counter electrode, and a second counter electrode. The working electrode, the reference electrode, the first counter electrode, and the second counter electrode are electrically isolated from each other.

### Advantageous Effects of Invention

According to the present disclosure, an electrochemical sensor that enables constant monitoring of water quality and enables highly accurate water quality monitoring.

### Brief Description of Drawings

FIG. 1 is a plan view illustrating the external appearance of an electrochemical sensor according to an embodiment.
FIG. 2 is an enlarged view of a main part of FIG. 1.
FIG. 3(a) is a cross-sectional view of the electrochemical sensor shown in FIG. 1 as viewed along the line IIIa-IIIa. FIG. 3(b) is a cross-sectional view of the electrochemical sensor shown in FIG. 1 as viewed along the line IIIb-IIIb.
FIG. 4(a) is a cross-sectional view showing an enlarged view of a working electrode in FIG. 3. FIG. 4(b) is a cross-sectional view showing an enlarged view of a reference electrode in FIG. 3.
FIG. 5 is a diagram showing the state between electrodes when the electrochemical sensor is immersed in the water to be inspected.
FIG. 6 is a diagram showing an equivalent circuit as an electrical circuit model occurring in the vicinity of the working electrode in the situation shown in FIG. 5.
FIG. 7 is a diagram for explaining a method for determining the resistance value of the charge transfer resistance and the capacitance of an electric double layer capacitor in the alternating current impedance method. The horizontal axis represents the real part, and the horizontal axis represents the imaginary part.
FIG. 8 is an example of a calibration curve showing the relationship between the resistance value of the charge transfer resistance and the pH of the water to be inspected.
FIG. 9 is an example of a calibration curve showing the relationship between the capacitance of an electric double layer capacitor and the total bacterial count of the water to be inspected.

### Description of Embodiments

In the following description, embodiments of an electrochemical sensor will be described in detail with reference to the attached drawings. The same elements will be assigned with the same reference numerals in the description of the drawings, and any overlapping description will not be repeated.

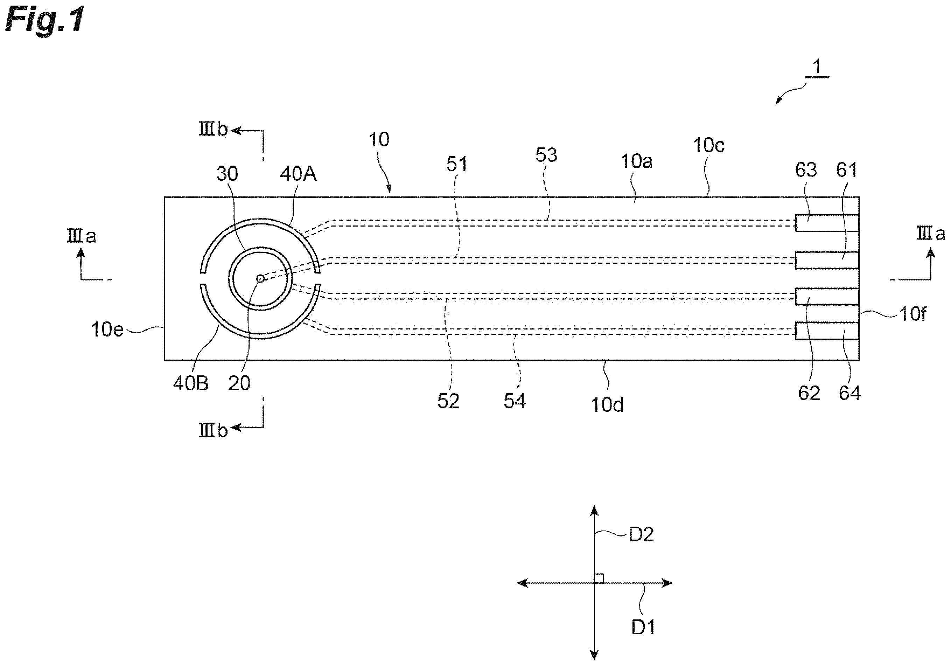

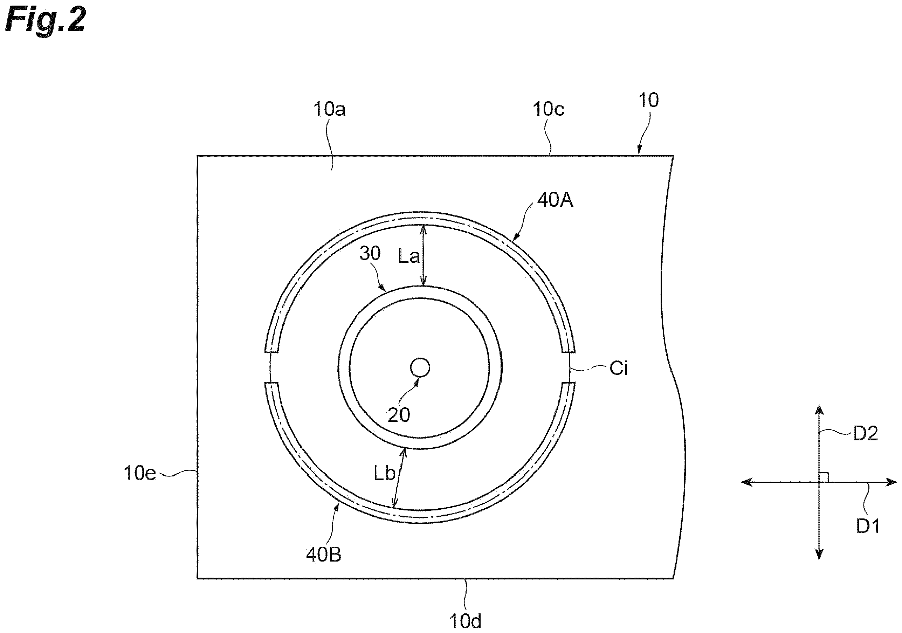

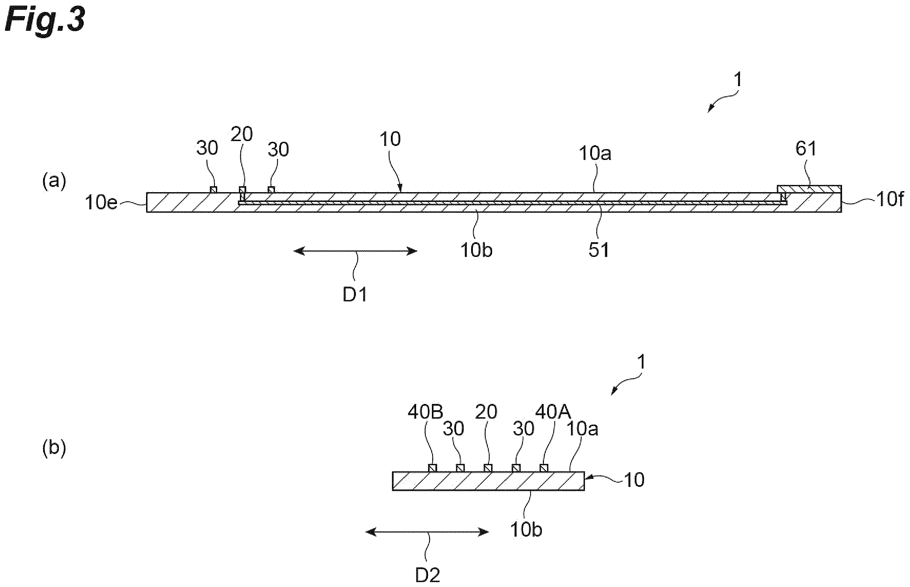

FIG. 1 is a plan view of an electrochemical sensor 1 according to an embodiment. FIG. 2 is an enlarged view of a main part of FIG. 1. FIG. 3(a) is a cross-sectional view of the electrochemical sensor 1 shown in FIG. 1 as viewed along the line IIIa-IIIa. FIG. 3(b) is a cross-sectional view of the electrochemical sensor 1 shown in FIG. 1 as viewed along the line IIIb-IIIb. The electrochemical sensor 1 is immersed in the water to be inspected for water quality inspection and outputs an electric signal indicating the water quality of the water to be inspected. The electric signal fluctuates according to the concentration of the substance to be detected. As shown in FIG. 1 and FIG. 2, the electrochemical sensor 1 of the present embodiment includes a dielectric substrate 10. Furthermore, the electrochemical sensor 1 includes a working electrode 20, a reference electrode 30, and counter electrodes 40A and 40B, which are respectively provided on a principal surface 10a of the dielectric substrate 10. The counter electrodes are also referred to as counter electrodes.

The planar shape of the dielectric substrate 10 is a rectangle having a certain direction D1 as a longitudinal direction. The dielectric substrate 10 has a flat principal surface 10a and a flat back surface 10b on the opposite of the principal surface 10a. The dielectric substrate 10 has a pair of lateral faces 10c and 10d extending along the direction D1 and being parallel to each other, and a pair of end faces 10e and 10f extending along the direction D2, which intersects (for example, orthogonally intersects) the direction D1, and being parallel to each other. The length of the dielectric substrate 10 in the direction D1 is, for example, 50 mm or more and 100 mm or less. The width of the dielectric substrate 10 in the direction D2 is, for example, 10 mm or more and 30 mm or less. The thickness of the dielectric substrate 10 is, for example, 2 mm or more and 10 mm or less. Examples of the constituent material of the dielectric substrate 10 include resins such as a thermosetting resin, a photocurable resin, and a thermoplastic resin. The dielectric substrate 10 may have flexibility as in the case of, for example, a flexible wiring board.

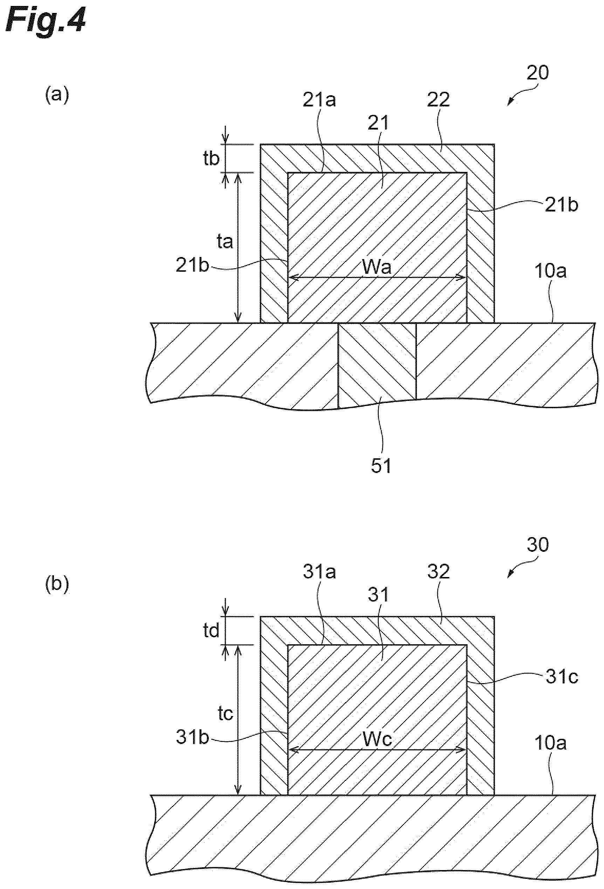

The working electrode 20 is a circular-shaped electrode. The working electrode 20 is provided at a position closer to one end, that is, the end face 10e, in the longitudinal direction of the dielectric substrate 10, that is, the direction D1. FIG. 4(a) is a cross-sectional view showing an enlarged view of the working electrode 20 in FIG. 3. As shown in FIG. 4(a), the working electrode 20 of the present embodiment has a base part 21 made of a metal and an electroconductive polymer film 22 covering the surface of the base part 21. In an example, the metal constituting the base part 21 is gold (Au). In that case, the base part 21 can be formed on the principal surface 10a by, for example, plating. Alternatively, the base part 21 may include at least one metal material selected from the group consisting of gold, platinum, and silver. The thickness ta of the base part 21 is, for example, 1 mm or more and 5 mm or less. The diameter Wa of the base part 21 is, for example, 1 mm or more and 5 mm or less. The base part 21 has a circular-shaped top face 21a along the principal surface 10a; and a lateral face 21b, which is a cylindrical surface.

The electroconductive polymer film 22 covers the top face 21a and the lateral face 21b of the base part 21. In an example, the electroconductive polymer film 22 is in close contact with the top face 21a and the lateral face 21b. In an example, the electroconductive polymer film 22 is a polyaniline film or a polypyrrole film. Alternatively, the electroconductive polymer film 22 may include at least one polymer material selected from the group consisting of polypyrrole, polyacetylene, poly(p-phenylene vinylene), polythiophene, polyaniline, and poly(p-phenylene sulfide). The electroconductive polymer film 22 can be formed by, for example, electrolytic polymerization. The thickness tb of the electroconductive polymer film 22 is, for example, 10 µm or more and 500 µm or less. In order to cover the surface of the base part 21 without gaps, the edge part of the electroconductive polymer film 22 may be in contact with the principal surface 10a over the entire circumference of the base part 21.

The reference electrode 30 is an annular-shaped electrode. The reference electrode 30 is formed so as to surround the circumference of the working electrode 20. The center of the reference electrode 30 coincides with the center of the working electrode 20. An annular-shaped gap is provided between the reference electrode 30 and the working electrode 20. FIG. 4(b) is a cross-sectional view showing an enlarged view of the reference electrode 30 in FIG. 3. As shown in FIG. 4(b), the reference electrode 30 of the present embodiment has a base part 31 made of a metal; and an electroconductive polymer film 32 covering the surface of the base part 31. In an example, the metal constituting the base part 31 is gold (Au). In that case, the base part 31 can be formed on the principal surface 10a by, for example, plating. Alternatively, the base part 31 may include at least one metal material selected from the group consisting of gold, platinum, and silver. The thickness tc of the base part 31 is, for example, 1 mm or more and 5 mm or less. The width Wc of the base part 31 is, for example, 1 mm or more and 5 mm or less. The base part 31 has an annular-shaped top face 31a along the principal surface 10a and a pair of lateral faces 31b and 31c. The lateral face 31b is the inner side surface of the base part 31. The lateral face 31c is the outer side surface of the base part 31.

The electroconductive polymer film 32 covers the top face 31a and the lateral faces 31b and 31c of the base part 31. In an example, the electroconductive polymer film 32 is in close contact with the top face 31a and the lateral faces 31b and 31c. In an example, the electroconductive polymer film 32 is a polyaniline film or a polypyrrole film. Alternatively, the electroconductive polymer film 32 may include at least one polymer material selected from the group consisting of polypyrrole, polyacetylene, poly(p-phenylene vinylene), polythiophene, polyaniline, and poly(p-phenylene sulfide). The electroconductive polymer film 32 can be formed by, for example, electrolytic polymerization. The thickness td of the electroconductive polymer film 32 is, for example, 10 µm or more and 500 µm or less. In order to cover the surface of the base part 31 without gaps, both of the edge part near the lateral face 31b and the edge part near the lateral face 31c of the electroconductive polymer film 32 may be in contact with the principal surface 10a over the entire circumference of the base part 31.

The counter electrode 40A is a first counter electrode according to the present embodiment. The counter electrode 40B is a second counter electrode according to the present embodiment. As shown in FIG. 2, the counter electrodes 40A and 40B are aligned to face each other in the transverse direction of the dielectric substrate 10, that is, the direction D2, with the working electrode 20 and the reference electrode 30 interposed between the counter electrodes 40A and 40B. The distance La between the counter electrode 40A and the reference electrode 30 is equal to the distance Lb between the counter electrode 40B and the reference electrode 30. In an example, the counter electrodes 40A and 40B extend along the circumferential direction of the reference electrode 30, and each of them has an arc shape concentric with the working electrode 20 and is located on a common circle Ci. Therefore, the reference electrode 30 and the counter electrodes 40A and 40B are disposed on concentric circles centered at the working electrode 20. In an example, the central angle and arc length of the counter electrode 40A are respectively equal to the central angle and the arc length of the counter electrode 40B.

The working electrode 20, the reference electrode 30, and the counter electrodes 40A and 40B are electrically isolated from each other. Here, being electrically isolated implies that the various electrodes are supported and fixed to each other only through a dielectric, and this means that the electrodes are substantially insulated from each other. In the present embodiment, the working electrode 20, the reference electrode 30, and the counter electrodes 40A and 40B are all fixed by means of the dielectric substrate 10, and in an unused state, the electrodes are adjacent to one another, with air interposed therebetween.

Regarding the control system of the electrochemical sensor 1, there are a potential control system and a current control system. In the potential control system, a potentiostat is used. The potentiostat applies a voltage between the working electrode 20 and the counter electrodes 40A and 40B and controls the potential between the working electrode 20 and the reference electrode 30 to a value that is wished to be set. Furthermore, in the current control system, a galvanostat is used. The galvanostat controls the current between the working electrode 20 and the counter electrodes 40A and 40B and measures the potential between the working electrode 20 and the reference electrode 30. As a control device, for example, HZ3000 manufactured by Hokuto Denko Corporation can be used.

As shown in FIG. 1, the electrochemical sensor 1 further includes four wiring patterns 51 to 54 and four terminals 61 to 64. The wiring patterns 51 to 54 are provided inside the dielectric substrate 10. The wiring patterns 51 to 54 extend linearly along the longitudinal direction of the dielectric substrate 10, that is, the direction D1. One end of the wiring pattern 51 is connected to the working electrode 20. One end of the wiring pattern 52 is connected to the reference electrode 30. One end of the wiring pattern 53 is connected to the counter electrode 40A. One end of the wiring pattern 54 is connected to the counter electrode 40B. In an example, the wiring patterns 51 to 54 are metal films provided between the layers of the dielectric substrate 10 that is formed by a plurality of dielectric layers laminated together.

The terminals 61 to 64 are an example of a connection part to be connected to a control device that performs potential control or current control in the electrochemical sensor 1. The terminals 61 to 64 are provided at the other end of the dielectric substrate 10, which is on the opposite of the one end of the dielectric substrate 10 where the working electrode 20, the reference electrode 30, and the counter electrodes 40A and 40B are provided. The terminals 61 to 64 are aligned along an end face 10f. The terminals 61 to 64 are metal films formed on the principal surface 10a or on the back surface 10b. The terminals 61 to 64 are exposed on the principal surface 10a or on the back surface 10b in order to enable electrical contact with connector terminals of the control device. The other ends of the wiring patterns 51 to 54 are connected to the terminals 61 to 64, respectively. The terminals 61 to 64 have a disposition and a shape that can be connected to a connector compliant with the Universal Serial Bus (USB) standard, that is, a USB connector, and the terminals 61 to 64 may be capable of being connected directly to a receptacle of a USB interface. Furthermore, the terminals 61 to 64 may also be capable of being connected to a control device such as a potentiostat holding a USB interface, directly or through a USB cable.

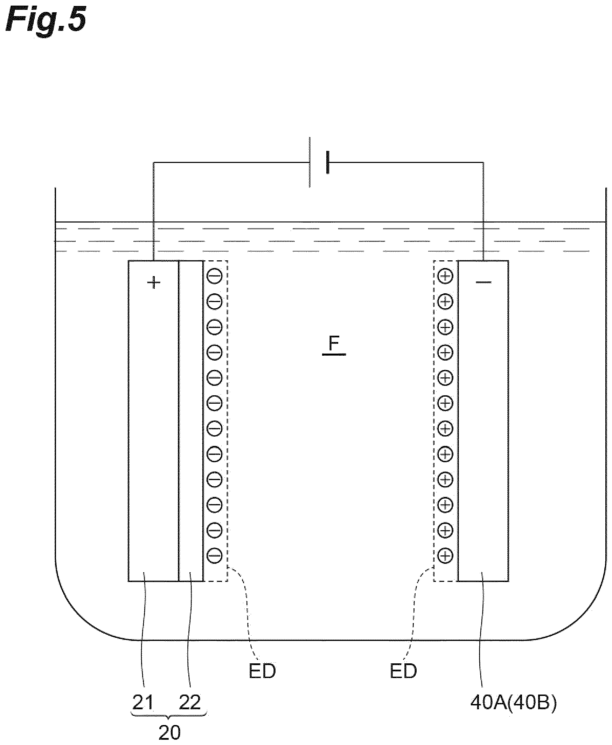

FIG. 5 is a diagram showing the state between electrodes when the electrochemical sensor 1 is immersed in water to be inspected F. As shown in FIG. 5, in the water of the water to be inspected F, an electric double layer ED is formed on the surface of each of the working electrode 20 and the counter electrodes 40A (40B). When an anode and a cathode are disposed in a system in which charged particles can move relatively freely, and a potential difference is applied, the charged particles move along the electric field. As a result, negatively charged particles (anions) are aligned in layers on the surface of the anode, and positively charged particles (cations) are aligned in layers on the surface of the cathode, to form electric double layers. In the electrochemical sensor 1, the working electrode 20 acts as an anode, and the counter electrode 40A (40B) acts as a cathode.

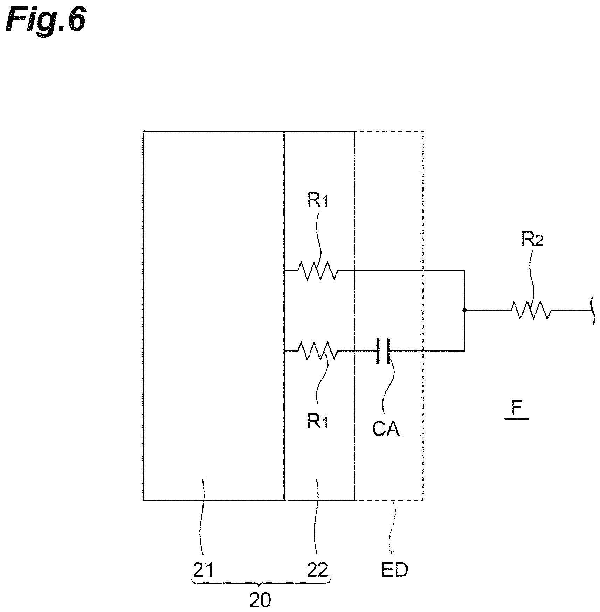

FIG. 6 is a diagram showing an equivalent circuit as an electric circuit model occurring in the vicinity of the working electrode 20 in the situation shown in FIG. 5. FIG. 6 shows a charge transfer resistance R₁, a solution resistance (reciprocal of conductance) R₂, and an electric double layer capacitor CA in the electric double layer ED. Among these, the resistance value of the charge transfer resistance R₁ corresponds to the oxidation-reduction rate and changes with the local hydrogen ion concentration (pH). Therefore, the pH of the water to be inspected F can be known by measuring the resistance value of the charge transfer resistance R₁ and then combining the value with a known calibration curve. Furthermore, bacteria have a number of structures mainly containing carboxylic acids on the surface, and the sizes of the bacteria are also large compared to other ions and the like. When such bacteria penetrate into the electric double layer ED, the capacitance of the electric double layer capacitor CA changes. Therefore, the total bacterial count (total bacterial quantity) of the water to be inspected F can be known by measuring the capacitance of the electric double layer capacitor CA and then combining the value with a known calibration curve.

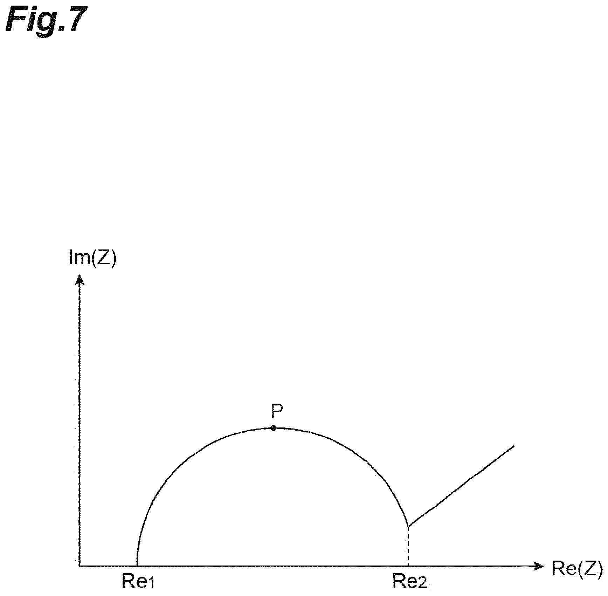

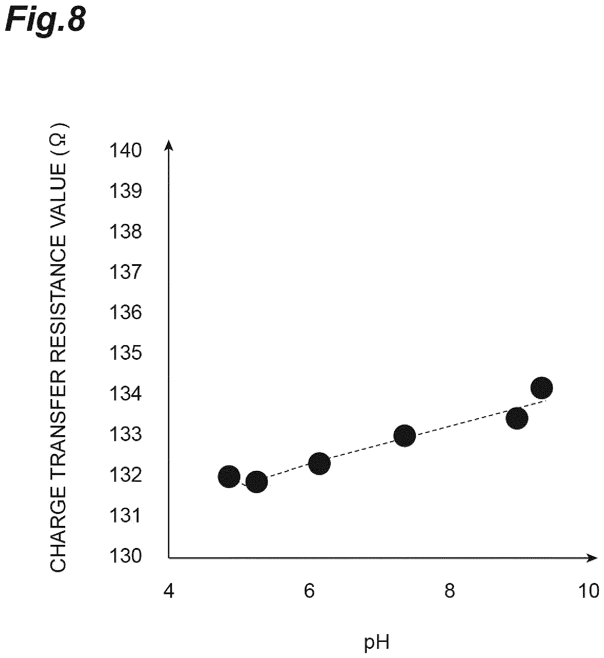

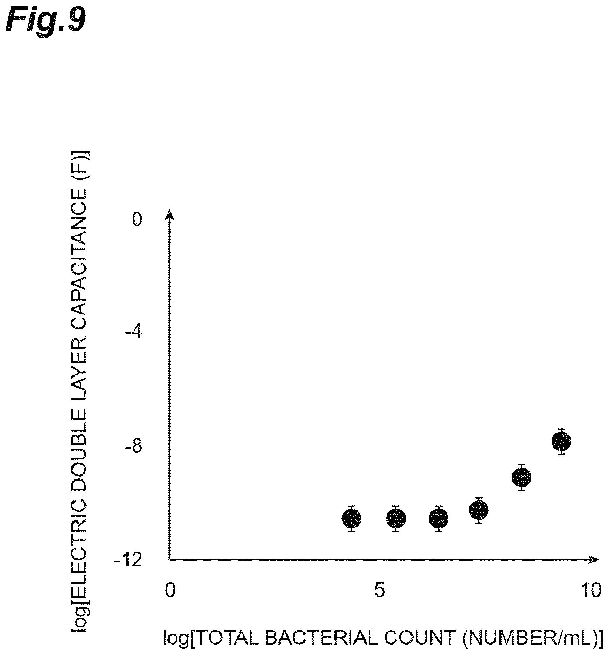

The resistance value of the charge transfer resistance R₁ and the capacitance of the electric double layer capacitor CA can be found by, for example, an alternating current impedance method. In the alternating current impedance method, an alternating current voltage is applied between the working electrode 20 and the counter electrode 40A (40B). Then, as shown in FIG. 7, the real number component and the imaginary number component of impedance are drawn on the complex plane to create a Nyquist diagram. In this case, a portion of the Nyquist diagram is approximately in a semicircular form. The real number component Re₁ at one end of the semicircle corresponds to the resistance value of the solution resistance R₂. The real number component Re₂ at the other end of the semicircle corresponds to the sum of the resistance value of the solution resistance R₂ and the resistance value of the charge transfer resistance R₁. Therefore, the resistance value of the charge transfer resistance R₁ can be found by determining the difference of these (Re₂ - Rei). Then, the pH of the water to be inspected F can be found from the resistance value of the charge transfer resistance R₁. Furthermore, the frequency corresponding to the apex P of the semicircular-shaped portion of the Nyquist diagram is correlated with the capacitance of the electric double layer capacitor CA. Therefore, the capacitance of the electric double layer capacitor CA can be found by determining the frequency corresponding to the apex P. Then, the total bacterial count of the water to be inspected F can be found from the capacitance of the electric double layer capacitor CA. In practice, in order to increase the measurement accuracy, an electrochemical impedance spectroscopy (EIS), which is an improved alternating current impedance method, or cyclic voltammetry (CV) or the like is used. FIG. 8 is an example of a calibration curve showing the relationship between the resistance value of the charge transfer resistance R₁ and the pH of the water to be inspected F. FIG. 9 is an example of a calibration curve showing the relationship between the capacitance of the electric double layer capacitor CA and the total bacterial count of the water to be inspected F.

Here, in the present embodiment, two counter electrodes 40A and 40B are provided. At the time of making measurement, the counter electrodes 40A and 40B are alternately switched and used at regular intervals. This alternate switching of the counter electrodes 40A and 40B at regular intervals can be carried out by, for example, simultaneously applying the same voltage to the counter electrodes 40A and 40B. Alternatively, this switching can be carried out by, for example, simultaneously applying a periodic voltage change to each of the counter electrodes 40A and 40B while shifting the phase. That is, the above-described measurement is performed between the working electrode 20 and the counter electrode 40A, subsequently or simultaneously the above-described measurement is performed between the working electrode 20 and the counter electrode 40B, and thereafter, measurement is repeated in the same manner. The measuring period is, for example, 10 milliseconds or more and 200 milliseconds or less.

The case of taking the pH and the total bacterial count of the water to be inspected as inspection items has been described above; however, inspection items such as the ion conductivity, Chemical Oxygen Demand (COD), the quantity of heavy metals, and the quantity of organic matter can also be similarly inspected by using the electrochemical sensor 1 according to the present embodiment. That is, the electrochemical sensor 1 of the present embodiment enables measuring of multiple items by using one sensor. According to the present embodiment, the water to be inspected is not particularly limited as long as it is water that enables inspection of the above-mentioned inspection items using the electrochemical sensor 1 of the present embodiment. Examples of the water to be inspected include tap water, sewage, and well water. According to the present embodiment, examples of water quality inspection include quantitative analysis, qualitative analysis, and semiquantitative analysis of the above-described inspection items in the water to be inspected. Constant monitoring of the water quality of the water to be inspected is enabled by immediate measurement of the above-mentioned inspection items. In the constant monitoring of the water quality of the water to be inspected, at least two inspection items among a plurality of the inspection items mentioned above may be immediately measured. The at least two inspection items can be appropriately selected from among the plurality of inspection items.

The effects obtainable by the electrochemical sensor 1 according to the present embodiment, which has been explained above, will be described. Usually, in the measurement by means of an electrochemical sensor, background noise caused by non-specific detection caused by unintended substances is mixed in the obtainable measurement data. Accordingly, there is a problem that the S/N ratio becomes small, and the measurement accuracy is deteriorated. Particularly, with regard to the measurement of bacteria such as Escherichia coli, conventional three-electrode electrochemical sensors have too small S/N ratios, and measurement results that can withstand practical use cannot be obtained. Currently, immediate measurement of the total bacterial count is not realized, and a method including culture which is time-consuming method as in the case of official methods is used.

With regard to such a problem, the electrochemical sensor 1 of the present embodiment includes two counter electrodes 40A and 40B, unlike conventional three-electrode type electrochemical sensors. Two different measurement data can be obtained by alternately switching the counter electrodes 40A and 40B or using them in parallel. The impedance between the working electrode 20 and the counter electrode 40A and the impedance between the working electrode 20 and the counter electrode 40B are different from each other; however, the difference between these two impedance values can be averaged by applying an electric field periodically or in parallel, and the background can be stabilized. Thus, highly accurate water quality monitoring is enabled. In addition, the measurement accuracy can be further enhanced by applying a periodic voltage change to each of the counter electrodes 40A and 40B while shifting the phase. From the above-described matters, a measurement accuracy sufficient for practical use can be obtained even for the measurement of bacteria such as Escherichia coli, by using the electrochemical sensor 1 of the present embodiment. Thus, it is possible to obtain the inspection results for water quality more rapidly as compared to methods including culturing. Therefore, constant monitoring of the water quality is enabled by using the electrochemical sensor 1 of the present embodiment.

In the present embodiment, the working electrode 20, the reference electrode 30, and the counter electrodes 40A and 40B are electrically isolated from each other. As a result, measurement by the working electrode 20, the reference electrode 30, and the counter electrode 40A and measurement by the working electrode 20, the reference electrode 30, and the counter electrode 40B can be each independently carried out.

In the water quality measurement according to the present embodiment, the noise intruding from the outside greatly affects the measurement accuracy. Particularly, there is a high risk that noise may be superimposed between the electrochemical sensor 1 and a control device such as a potentiostat. As in the present embodiment, the noise superimposed between the electrochemical sensor 1 and the control device can be reduced by adjusting the terminals 61 to 64 of the electrochemical sensor 1 to a disposition and a shape that can be connected to connectors compliant to the noise-resistant USB standard.

As in the present embodiment, the reference electrode 30 may exhibit an annular shape surrounding the circumference of the working electrode 20. In that case, the counter electrodes 40A and 40B may exhibit an arc shape extending along the circumferential direction of the reference electrode 30. In this case, a configuration in which the reference electrode 30 is disposed between the counter electrodes 40A and 40B and the working electrode 20 can be easily realized.

As in the case of the present embodiment, the counter electrodes 40A and 40B may be located on a common circle Ci. In this case, the distance La between the counter electrode 40A and the reference electrode 30 can be easily made equal to the distance Lb between the counter electrode 40B and the reference electrode 30.

As in the case of the present embodiment, the working electrode 20 may include a base part 21 made of a metal; and an electroconductive polymer film 22, for example, a polypyrrole film, covering the surface of the base part 21. When the surface of the metal electrode is exposed without disposing the electroconductive polymer film 22, there is a risk that non-specific components such as ion components may inhibit normal detection. When the electroconductive polymer film 22 is disposed, non-specific behavior can be suppressed, and the measurement accuracy of each inspection item can be further increased.

As in the case of the present embodiment, the reference electrode 30 may include a base part 31 made of a metal; and an electroconductive polymer film 32, for example, a polyaniline film or a polypyrrole film, covering the surface of the base part 31. When the electroconductive polymer film 32 is applied, the electrochemical behavior of water can be stabilized. That is, when there is only the surface of the metal electrode, the behavior of non-specific components such as ion components is conducted at the same time as the electrochemical behavior of water, and there is a risk that the function as the reference electrode may be impaired. When the electroconductive polymer film 32 is disposed, since non-specific behavior can be suppressed, and the reference electrode can function more effectively, the measurement accuracy of each inspection items can be further increased.

The electrochemical sensor according to the present disclosure is not limited to the above-mentioned embodiment, and various other modifications can be made. For example, each of the above-mentioned modification examples may be combined according to the required purpose and effects. In the above-described embodiment, a case in which two counter electrodes 40A and 40B are provided has been described as an example, it is also acceptable to provide three or more counter electrodes. Even in that case, effects similar to the above-described embodiment can be provided. In the above-described embodiment, an alternating current impedance method, EIS, and CV have been mentioned as examples of the measurement method. The measurement method is not limited to these, and for example, other measurement methods such as a constant potential measurement method may be used.

### Reference Signs List

1: electrochemical sensor, 10: dielectric substrate, 10a: principal surface, 10b: back surface, 10c, 10d: lateral face, 10e, 10f: end face, 20: working electrode, 21: base part, 21a: top face, 21b: lateral face, 22: electroconductive polymer film, 30: reference electrode, 31: base part, 31a: top face, 31b, 31c: lateral face, 32: electroconductive polymer film, 40A, 40B: counter electrode, 51 to 54: wiring pattern, 61 to 64: terminal, CA: electric double layer capacity, Ci: circle, D1, D2: direction, ED: electric double layer, F: water to be inspected, R₁: charge transfer resistance, R₂: solution resistance.

## Claims

1. An electrochemical sensor used in a state of being immersed in water to be inspected for water quality inspection, the electrochemical sensor comprising:
a working electrode;
a reference electrode;
a first counter electrode; and
a second counter electrode,
wherein the working electrode, the reference electrode, the first counter electrode, and the second counter electrode are electrically isolated from each other.

2. The electrochemical sensor according to claim 1, wherein the reference electrode is disposed between both of the first counter electrode and the second counter electrode, and the working electrode, and
wherein the first counter electrode and the second counter electrode are disposed to face each other, with the working electrode and the reference electrode interposed therebetween.

3. The electrochemical sensor according to claim 2, wherein the reference electrode exhibits an annular shape surrounding a circumference of the working electrode, and
wherein the first counter electrode and the second counter electrode exhibit an arc shape extending along a circumferential direction of the reference electrode.

4. The electrochemical sensor according to claim 3, wherein the first counter electrode and the second counter electrode are located on a common circle.

5. The electrochemical sensor according to claim 4, wherein the reference electrode, the first counter electrode and the second counter electrode are disposed on concentric circles centered at the working electrode.

6. The electrochemical sensor according to any one of claims 1 to 5, wherein each of the working electrode and the reference electrode comprises a base part made of a metal; and an electroconductive polymer film covering a surface of the base part.

7. The electrochemical sensor according to claim 6, wherein the electroconductive polymer film is a polypyrrole film or a polyaniline film.

8. The electrochemical sensor according to any one of claims 1 to 7, further comprising a connection part to be connected to a control device conducting potential control or current control of the electrochemical sensor.

9. The electrochemical sensor according to claim 8, wherein the connection part is configured to be connectable to a USB connector.
